# EUROPEAN PATENT APPLICATION

(11) **EP 2 769 741 A1**
(43) Date of publication of application: **27.08.2014**
(21) Application number: 13156274.6
(22) Date of filing: 22.02.2013
(51) Int. Cl.: A61L 27/28, A61L 31/08

(54) **Medical device with a biocompatible coating**

(71) Applicant: Cardiatis S.A., 5032 Isnes (BE); Lylyk, Pedro, C1428ARJ Buenos Aires (AR)
(72) Inventor: Lylyk, Pedro, C1428ARJ Buenos Aires (AR); Frid, Noureddine, 1650 Beersel (BE); Köntges, Nils, 1180 Brussels (BE); Gebhart, Laurence, 1084 Brussels (BE)
(74) Representative: Delcoux, Mariette

(57) **Abstract**

An implantable medical device comprising (a) a metallic substrate and (b) a bisphosphonate wherein both phosphorus atoms contained in the bisphosphonate are covalently attached to a same carbon atom. The bisphosphonate continuously coats the external surface of the metallic substrate as monolayer and as outermost layer. At least one phosphonate moiety of the bisphosphonate is covalently and directly bonded to the external surface of the metallic substrate and/or covalently bonded to another molecule of the bisphosphonate in the coating.

## Description

### Technical Field

The present invention relates to a biocompatible coating for an implantable medical device having a metallic part, and more particularly relates to a bisphosphonate-based coating covalently and directly bonded to a surface of the metallic part. This invention also relates a method for producing an implantable device having a metallic part to which a bisphosphonic acid compound is covalently and directly bonded as external coating.

### Background of the invention

Implantable medical devices such as artificial heart valves, vascular prosthesis, stents, orthopedic screws and joint prosthesis often comprise or essentially consist of a metallic substrate to obtain adequate mechanical properties. However, the metallic substrates used for implantable medical devices have disadvantage with regard to their biocompatibility or functionality, in particular, in long-term use.

Implants often trigger inflammatory tissue responses and immune reactions through chemical and/or physical irritation, thus resulting in intolerance reactions in the sense of chronic inflammatory reactions with defensive and rejection reactions, excessive scar tissue production of degradation of tissue. There has therefore been a demand for a coating for the implants that reduces inflammatory responses caused by implantation.

Stents, in general, are endovascular prostheses or implants which are used i.a. for treating stenosis. (Re)stenosis is a narrowing of a blood vessel, leading to restricted blood flow, blockage of the blood and insufficient oxygenation of cardiac tissue, resulting in myocardial ischemia, infarction, cardiac arrhythmia, or cardiac arrest. Basically, stents have a carrier structure suitable for supporting the wall of a vessel in an appropriate manner in order to enlarge the vessel. Restenosis often occurs after a first operation for treating stenosis has been carried out (for example, dilatation of a vessel by a balloon or a classical stent).

U.S. Pat. No. 5741333 discloses a self-expanding bare metal stent, a tiny metal scaffolding that functions to brace the vessel wall, which effectively reduces restenosis and significantly decreases the rates of the major adverse cardiac events, myocardial infarction, and death.

A stated above, stent implantation is often associated with in-stent restenosis, which is defined as late lumen diameter loss greater than 50% within the stent. In-stent restenosis is associated with an excessive proliferation of vascular smooth muscle cells (SMCs), extracellular matrix synthesis and a chronic inflammatory reaction which are believed to be initiated by a deep vascular injury and the endothelial cell denudation created under angioplasty and further by the presence of a foreign metallic device. This complication occurs in about 15 to 30% of the procedures.

Drug-eluting stents (DES), namely, stents combined with drugs, were put on the market to theoretically prevent in-stent restenosis phenomenon through inhibition of SMC proliferation. Generally, there are three components in DES, i.e., a stent platform, drug delivery vehicle such as polymer matrix, and drug such as sirolimus and paclitaxel. Such DESs are supposed to significantly reduce the rate of restenosis compared to bare metal stent. However, an increase in the rate of myocardial infarction and cardiovascular mortality was reported after the implantation of such DES. These events were found to be due to late stent thrombosis, which is often carried out after an acute thrombosis of the artery caused by the platelet aggregation and blood clotting.

In order to alleviate thrombosis and in-stent restenosis, there are various approaches in the state of art for improving the biocompatibility of stents by modifying their surfaces with less thrombotic and inflammatory materials. U.S. Pat. No. 5,891,507, for example, discloses metal stents coated with silicone, polyetrafluoroethylene and biological materials such as heparin or growth factors which increase the biocompatibility of the metal stents.

### Summary of the invention

The present invention provides an implantable medical device having a new type of coating on a metallic surface thereof.

The coating according to the present invention comprises a bisphosphonic acid which is covalently and directly bonded to a metallic surface of an implantable medical device and forms a monolayer as external layer of the coating. Some molecules of the bisphosphonic acid may be covalently bonded to another molecule of the bisphosphonic acid in the coating. The coating provides biocompatibility for the metallic surface, resulting in reducing inflammatory responses caused by the implantation. The coating also provides improved platelet-antiadhesion property for the metallic sureface while keeping good compatibility with the platelet poor plasma (PPP) and with the endothelial cells which are required for rapid endothelialization on the coating. As a result, the risk of late thrombosis and in-stent restenosis can be reduced with the coating.

The object of the invention is to put on the market an endoprosthesis that provides increased biocompatibility of its surface, resulting in reducing the risk of a chronic inflammatory reactions, thrombosis, and in-stent restenosis, caused by the implantation.

The subject of the present invention is defined in the appended independent claims. Preferred embodiments are defined in the dependent claims.

The subject of the present invention is an implantable medical device comprising a metallic substrate and a bisphosphonate. Both phosphorus atom contained in the bisphosphonate are covalently attached to a same carbon atom. The bisphosphonate continuously coats the external surface of the metallic substrate as monolayer and as outermost layer, and at least one phosphonate moiety of a molecule of the bisphosphonate is covalently and directly bonded to the external surface of the metallic substrate and/or covalently bonded to another molecule of the bisphosphonate in the coating.

Another subject of the present invention is an implantable medical device comprising a metallic substrate and a bisphosphonate. The bisphosphonate may consist of the general formula (I). 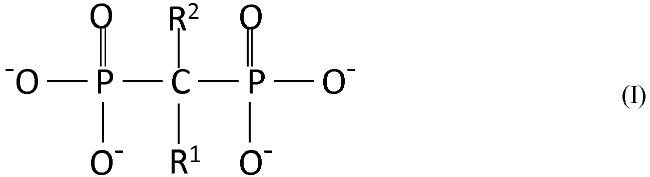
R¹ may represent:
   (i) -C₁₋₅ alkyl, unsubstituted or substituted with -NH₂, pyridyl, pyrrolidyl or - NR³R⁴;
   (ii) -NHR⁵;
   (iii) -SR⁶; or
   (iv) -Cl;
R² may represent -H, -OH, or -Cl;
R³ may represent -H or -C₁₋₅ alkyl;
R⁴ may represent -C₁₋₅ alkyl;
R⁵ may represent -C₁₋₁₀ alkyl or -C₃₋₁₀ cycloalkyl;
R⁶ may represent phenyl.

In another embodiment, the surface phosphorus-atom concentration of the coating of the bisphosphonate substrate is at least 70%P, preferably at least 80 %P.

In another embodiment, the implantable medical device can be selected from the group consisting of vascular endoprosthesis, intraluminal endoprosthesis, stents, coronary stents and peripheral stents.

In another aspect, the present invention provides a method for coating a metallic substrate with a bisphosphonate substrate, comprising the following steps:
(a) providing the metallic substrate;
(b) preparing a liquid carrier comprising a bisphosphonic acid;
(c) immersing the metallic substrate into the liquid carrier; and
(d) covalently immobilizing the bisphosphonic acid compound onto a surface of the metallic substrate in the liquid carrier, and both phosphorus atom contained in the bisphosphonic acid are covalently attached to same carbon atom.

In one embodiment of the invention, the bisphosphonic acid having the formula (I) or a salt thereof, wherein: 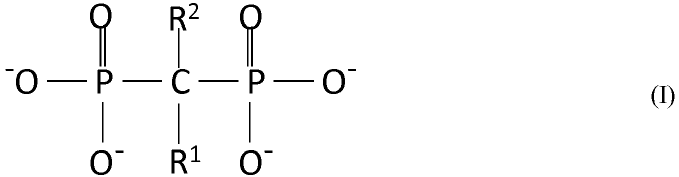
R¹ may represent:
   (i) -C₁₋₅ alkyl, unsubstituted or substituted with -NH₂, pyridyl, pyrrolidyl or - NR³R⁴;
   (ii) -NHR⁵;
   (iii) -SR⁶; or
   (iv)-Cl;
R² may represent -H, -OH, or -Cl;
R³ may represent -H or -C₁₋₅ alkyl;
R⁴ may represent -C₁₋₅ alkyl;
R⁵ may represent -C₁₋₁₀ alkyl or -C₃₋₁₀ cycloalkyl;
R⁶ may represent phenyl.

In another embodiment, the liquid carrier may be heated for at least 24 hours, preferably 48 hours, at a temperature at least 70 °C, preferably at least 80 °C in step (d).

In another embodiment, the metallic substrate in the liquid carrier is subjected to an induction heating in step (d).

Still another embodiment of the invention relates the method, wherein the surface of the metallic substrate to be coated can be subjected to a thermal treatment for promoting the formation of a thick external metal oxide layer thereon, preferably, the thermal treatment comprises a step of heating the metallic substrate to be coated for at least 10 minutes, preferably at least 15 minutes, at a temperature at least 500°C, preferably at least 550°C.

Another subject of the present invention relates to a bisphosphonate for use as anti-inflammatory coating for a metallic surface of an implantable medical device, wherein both phosphorus atoms of the bishosphonate are covalently attached to same carbon atom.

Still another subject of the present invention relates to a bisphosphonate for use as anti-thrombogenic coating for a metallic surface of a endovascular prostheses, wherein both phosphorus atoms of the bishosphonate are covalently attached to same carbon atom.

### Brief description of the Figures

Figure 1 is comparative graph showing platelet rich plasma (PRP) adhesion on a bare-metal surface as comparative example (CEX) and on a bisphosphonate coating according to the invention (INV).
Figure 2 is comparative graph showing platelet poor plasma (PPP) adhesion on a bare-metal surface as comparative example (CEX) and on a bisphosphonate coating according to the invention (INV).
Figure 3 is comparative graph showing relative endothelialisation on a bare-metal surface as comparative example (CEX) and on a bisphosphonate coating according to the invention (INV).
Figure 4 is comparative graph showing relative inflammation with a bare-metal surface as comparative example (CEX) and with a bisphosphonate coating according to the invention (INV).

### Detailed description of the invention

The terms "implantable medical device" and "implant" are used synonymously here and are understood to include medical or therapeutic implants, such as vascular endoprosthesis, intraluminal endoprosthesis, stents, coronary stents, peripheral stents, surgical and/or orthopedic implant for temporary use, such as surgical screws, plates, nails and other fastening means, permanent surgical or orthopedic implants, such as bone prosthesis or joint prosthesis.

The implantable medical device according to the present invention comprises, or essentially consists of, a metallic substrate which is selected from the group consisting of iron, magnesium, nickel, tungsten, titanium, zirconium, niobium, tantalum, zinc or silicon and, if necessary, a second component of one or several metals from the group consisting of lithium, sodium, potassium, calcium, manganese, iron or tungsten, preferably of a zinc-calcium alloy. In a further practical example, the metallic substrate consists of a memory effect material of one or several materials from the group consisting of nickel titanium alloys and copper zinc aluminium alloys, but preferably of nitinol. In a further practical example, the metallic substrate of the medical device consists of stainless steel, preferably of a Cr-Ni-Fe steel, in this case, preferably the alloy 316L, or a Co-Cr steel such as Phynox. In preferred embodiments of the present invention, the implantable medical devices are stents, in particular metal stents, preferably self-expanding stents.

This invention is related to a coating for a metallic substrate comprising a bisphosphonate layer derived from a bisphosphonic acid, the two phosphorus atoms of the bisphosphonic acid are covalently attached to same carbon atom.

U.S. Pat. No. 5,431,920 discloses a composition in dosage form comprising a bisphosphonic acid as active ingredient for treating metabolic bone diseases such as osteoporosis.

European Pat No. 1,508,343 discloses a multilayer coating for an implant device for use of bone fixation. The coating comprises two types of bisphosphate layers; one is designed to release from the multilayer coating immediately after the implant device has been inserted and the other designed slowly released over time for the long-time use of the implant device. The latter type of bisphosphate layer is strongly bonded to a plurality of protein layers which are immobilized on a surface of the implant device. The former type of bisphosphate is loosely bonded to the latter type of layers as outermost layer of the coating.

According to the present invention, a bisphosphonate coats a metallic surface of an implantable medical device as monolayer and as outermost layer. At least one phosphonate moiety of the bisphosphonate is covalently and directly bonded to the metallic surface. Some molecules of the bisphosphonate may covalently bond to another molecule of the bisphosphonate in the coating. Therefore, after implantation, the bisphosphonate or bisphosphonic acid is not released by the coating as an active ingredient but permanently stays on the surface as a part of the coating. Surprisingly, the mere presence of the bisphosphonate layer provides biocompatibility to the coating and reduces the inflammatory response and/or promotes the formation of an endothelial cell layer on the coating while significantly decreasing the activation and adhesion of platelet. As a result, the risks of restenosis and thrombosis are strongly reduced.

"Phosphate group" means a functional group comprising phosphorus attached to four oxygens and with net negative charge, thus presented as PO₄⁻. "Phosphonic acid" is an organic compound containing C-PO(OH)₂ group. "Phosphonate" is a salt or ester of an phosphonic acid and has a general formula R¹-PO(OR²)₂ group (where R¹ represents alkyl or aryl and R² represents alkyl, aryl or a counter cation of the salts). For purposes of the present invention, "bisphosphonate" refers to compounds having two phosphonate (PO₃) groups in the molecule. Bisphosphonates according to the present invention have a common P-C-P "backbone", namely, the two phosphonate (PO₃) groups covalently linked to one carbon atom.

In preferred embodiments of the present invention, a bisphosphonic acid used for providing the bisphosphonate monolayer on the metallic surface of the implantable medical device has the general formula (I) or a salt thereof.

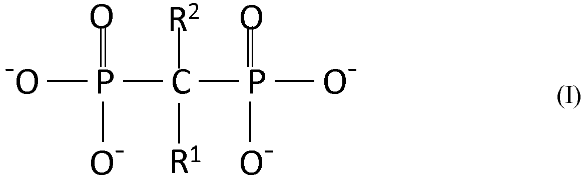

In the formula (I) R¹ represents -C₁₋₅ alkyl (which is unsubstituted or substituted with -NH₂, pyridyl, pyrrolidyl or -NR³R⁴), -NHR⁵, -SR⁶ or -Cl; R² represents -H, -OH, or -Cl; R³ represents -H or -C₁₋₅ alkyl; R⁴ represents -C₁₋₅ alkyl; R⁵ represents -C₁₋₁₀ alkyl or -C₃₋₁₀ cycloalkyl; R⁶ represents phenyl.

As used herein, "alkyl" is intended to include both branched- and straight-chain saturated aliphatic hydro carbon groups having the specified number of carbon atoms; "cycloalkyl" is intended to include saturated ring groups, such as cyclopropyl, cyclobutyl, cyclopentyl, cyclohexyl and cycloheptyl.

In preferred embodiments of the present invention, the bisphosphonic acid can be selected from the group consisting of 1-hydroxyethylidene-1,1-diphosphonic acid (etidronic acid), alendronic acid, clodronic acid, pamidronic acid, tiludronic acid and risedronic acid, preferably 1-hydroxyethylidene-1,1-diphosphonic acid (etidronic acid).

The bisphosphonate in the coating according to the present invention continuously coats the external surface of the metallic substrate as monolayer and as outermost layer, and at least one phosphonate moiety of each molecule of the bisphosphonate is covalently and directly bonded to the external surface of the metallic substrate and/or covalently bonded to another molecule of the bisphosphonate in the coating.

Induction heating is a widely used method to heat metallic substrates directly and contactless by electromagnetic induction, where eddy currents are generated within the metal and resistance leads to Joule heating of the metal. An induction heater (for any process) consists of an electromagnet such as coil, through which a high-frequency alternating current (AC) is passed.

In a preferred embodiment, a container made of non-conductive material comprising a metallic substrate to be coated and a liquid carrier containing the bisphosphonic acid compound is located inside of a conductive coil. The metallic substrate is subj ect to the eddy current produced by passing through the coil a AC with a power output of between 50 and 500 kW and a frequency between 150 and 250 kHz, so as to obtain a bisphosphate layer on a surface of the metallic substrate with a desirable thickness. The concentration of the bisphosphonic acid compound is between 10⁻² and 10⁻⁴ mol/l, preferably 10⁻³ mol/l. The bisphosphonic acid compound is preferably dissolved in the liquid carrier. The liquid carrier may be alcohol such as methanol and ethanol and isopropanol, THF, DMF, water or a mixture thereof.

In a further preferred embodiment, a metallic substrate to be coated is immersed in a liquid carrier containing the bisphosphonic acid at a concentration between 10⁻² and 10⁻⁴ M and the liquid carrier is heated by conventional means for at least 24 h at a temperature of at least 70 °C.

The metallic substrate may be subject to a thermal treatment (TT) so as to promote the formation of a thick oxidized layer on the metallic substrate.

Surface compositions (total atomic concentration %) of bisphosphate monolayers on a metallic substrate has been measured with X-ray photoelectron spectroscopy (XPS). The surface phosphorus-atom concentration of the coating of the bisphosphate substrate is at least 70 %P, preferably at least 90 %P.

### Examples

### Example 1: (formation of a coating)

5 mm Phynox stents in 2 cm long were provided as medical device to be coated. The Phynox surface was modified by a thermal treatment (TT) in an oven for 15 min at 550 °C, and then a chemical treatment (CT, immersing the stent into a solution of 0.1% HF and 20% HNO₃ for 15 min and then immersing the stent in solution of 2.5% HNO₃ for 30 min) was performed. The stents were subjected to an ethylene oxide sterilization process before surface treatment. Etidronic acid (EA) aqueous solution was obtained by dilution 1000x of the mother solution (60%v/v). The stent was immersed into the solution and heated at 80 °C for 48 hours.

### Example 2: (induction heating)

5 mm Phynox stents in 2 cm long stent were provided as medical device to be coated. The Phynox surface was modified by a thermal treatment (TT) in an oven for 15 min at 550 °C, and then a chemical treatment (CT, immersing the stent into a solution of 0.1% HF and 20% HN03 for 15 min and then immersing the stent in solution of 2.5% HNO₃ for 30 min) was performed. Etidronic acid aqueous solution was prepared at a concentration of 10⁻³ M. The stents were immersed into the solution and subjected to the induction heating with Ambrell EasyHeat induction heating system with a power output of 110 kW and a frequency of 198 kHz. The used solenoid was composed of 7 spires with an internal diameter of 9 cm.

### Example 3: (Platelet adhesion)

Blood was collected in 3.6ml sodium citrate 3.5% vials to prevent coagulation. Full blood was being centrifuged at two different rotation speeds in order to obtain two different types of plasma. The first type (a plasma rich in platelets (PRP)) was obtained by centrifugation at 900 rpm, and the second type (a plasma poor in platelets (PPP)) was obtained by centrifugation at 1500 rpm. In order to have sufficient volume for the test, plasma was diluted in HBSS (Hank's buffered salt solution). Platelets adhesion was assessed by spectrophotometry. The amount of total protein was evaluated by measurement of the absorbance at 280 nm. The bare-metal stents and the ones with the EA coating were prepared as described in example 1 and immersed in one of the two plasma solutions for 24 hours, at 37°C and under constant agitation (STAT-FAX 2200 Thermostated agitator). When completed the stents are rinsed with HBSS buffer and then treated with 125 µl lysis solution. Total absorbance at 280 nm is than being measured. Raw data were normalized by dividing every sample value by the average of the control for every experiment (PRP and PPP).

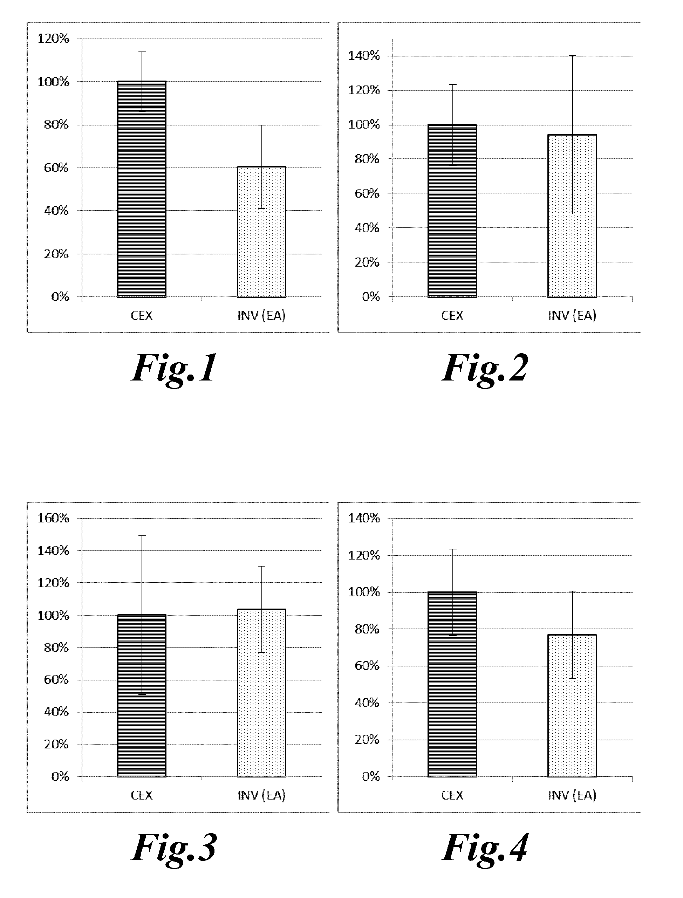

Figs. 1 and 2 show respectively PRP and PPP adhesions to the bare-metal surface (CEX) and to the EA coating according to the invention (INV). The difference between the PRP and the PPP value reflects the amount of platelets adsorbed on the surface.

Conclusion: An important (40% !) decrease of platelet aggregation was observed on the EA coating according to the invention compared to the bare-metal surface.

### Example 4: (Endothelial cells growth: endothelialisation)

The bare-metal stents and the ones with the EA coating were prepared as described in example 1 and incubated with 70.000 cells (EA.hY926) per well for 7 days (37°C, CO₂ 5%). This large amount of cells was meant to enhance the probability for cells to be trapped into the meshing of the stents. 80% (4ml out of 5ml) of the culture medium was replaced twice during the incubation period (after 1 and 4 days). When completed the stents are rinsed with HBSS buffer and then treated with 125 µl lysis solution. Total absorbance at 280 nm is than being measured.

Figure 3 shows relative endothelialisation on the bare-metal surface (CEX) and on the EA coating according to the invention (INV).

Conclusion: a same level of endothelial cells growth was observed on the EA coating as compared to the bare-metal surface. Thus, desirable ability of wall cell regeneration is not reduced while decreasing platelet aggregation.

### Example 5: (Inflammation induction)

The IL-8 values obtained with the bare-metal stents and with the EA coating stents prepared as described in example 1 were measured in the medium using a regular ELISA set in accordance with the instructions provided.

Figure 4 shows relative inflammation with the bare-metal surface (CEX) and with the EA coating according to the invention (INV).

Conclusion: The inflammatory response induced with the EA coating was significantly improved, i.e., 23% lower, compared to the bare-metal surface.

Accordingly, the bisphosphonate coating according to the present invention exhibited a significant decease of platelet adhesion and cell inflammatory response compared to the bare-metal surface while keeping rapid promotion of endothelial cell growth thereon.

## Claims

1. An implantable medical device comprising:
(a) a metallic substrate; and
(b) a bisphosphonate,
**characterized in that** both phosphorus atoms contained in the bisphosphonate are covalently attached to a same carbon atom, the bisphosphonate continuously coating the external surface of the metallic substrate as monolayer and as outermost layer, and at least one phosphonate moiety of the bisphosphonate being covalently and directly bonded to the external surface of the metallic substrate and/or covalently bonded to another molecule of the bisphosphonate in the coating.

2. The implantable medical device according to claim 1, wherein the bisphosphonate has the general formula (I), 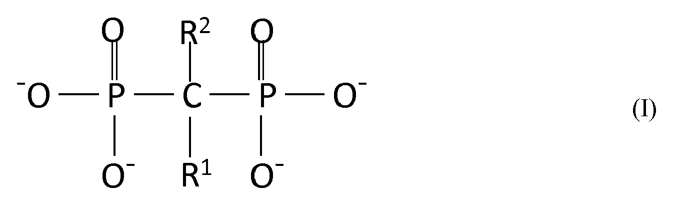 wherein:
R¹ represents:
(i) -C₁₋₅ alkyl, unsubstituted or substituted with:
• -NH₂;
• pyridyl;
• pyrrolidyl; or
• -NR³R⁴
(ii) -NHR⁵;
(iii) -SR⁶; or
(iv)-Cl;
R² represents -H, -OH, or -Cl;
R³ represents -H or -C₁₋₅ alkyl;
R⁴ represents -C₁₋₅ alkyl;
R⁵ represents -C₁₋₁₀ alkyl or -C₃₋₁₀ cycloalkyl;
R⁶ represents phenyl, and

3. The implantable medical device according to claim 2, wherein R¹ represents - CH₃ and R² represents -OH.

4. The implantable medical device according to any one of preceding claims, wherein the surface phosphorus-atom concentration of the coating of the bisphosphonate substrate is at least70%P, preferably at least 80 %P.

5. The implantable medical devices according to any one of preceding claims, selected from the group consisting of vascular endoprosthesis, intraluminal endoprosthesis, stents, coronary stents and peripheral stents.

6. A method for coating a metallic substrate with a bisphosphonate substrate, comprising the following steps:
(a) providing the metallic substrate;
(b) preparing a liquid carrier comprising a bisphosphonic acid or a salt thereof;
(c) immersing the metallic substrate into the liquid carrier; and
(d) covalently immobilizing the bisphosphonic acid compound onto a surface of the metallic substrate in the liquid carrier,
**characterized in that** both phosphorus atom contained in the bisphosphonic acid are covalently attached to same carbon atom.

7. The method according to claim 6, wherein the bisphosphonic acid having the formula (I), wherein: 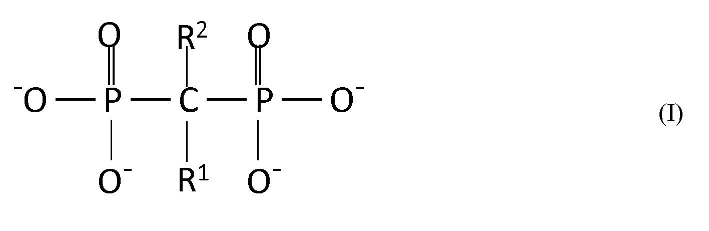
R¹ represents:
(i) -C₁₋₅ alkyl, unsubstituted or substituted with:
• -NH₂;
• pyridyl;
• pyrrolidyl; or
• NR³R⁴
(ii) -NHR⁵;
(iii) -SR⁶; or
(iv) -Cl;
R² represents -H, -OH, or -Cl;
R³ represents -H or -C₁₋₅ alkyl;
R⁴ represents -C₁₋₅ alkyl;
R⁵ represents -C₁₋₁₀ alkyl or -C₃₋₁₀ cycloalkyl;
R⁶ represents phenyl.

8. The method according to claim 7, wherein the bisphosphonic acid is selected from the group consisting of 1-hydroxyethylidene-1,1-diphosphonic acid (etidronic acid), alendronic acid, clodronic acid, pamidronic acid, tiludronic acid and risedronic acid, preferably etidronic acid.

9. The method according to any one of claims 6 to 8, wherein the liquid carrier is heated for at least 24 hours, preferably 48 hours, at a temperature at least 70 °C, preferably at least 80 °C in step (d).

10. The method according to any one of claims 6 to 8, wherein the metallic substrate in the liquid carrier is subjected to an induction heating in step (d).

11. The method according to any one of claims 6 to 10, wherein the surface of the metallic substrate to be coated is subjected to a thermal treatment for promoting the formation of a thick external metal oxide layer thereon.

12. The method according to claim 11, wherein the thermal treatment comprises a step of heating the metallic substrate to be coated for at least 10 minutes, preferably at least 15 minutes, at a temperature at least 500°C, preferably at least 550°C.

13. Bisphosphonate for use as anti-inflammatory coating for a metallic surface of an implantable medical device, wherein both phosphorus atoms of the bishosphonate are covalently attached to same carbon atom.

14. Bisphosphonate for use as anti-thrombogenic coating for a metallic surface of an endovascular prosthesis, wherein both phosphorus atoms of the bishosphonate are covalently attached to same carbon atom.
